# EUROPEAN PATENT SPECIFICATION

(11) **EP 3 166 511 B1**
(45) Date of publication and mention of the grant of the patent: **02.05.2018**
(21) Application number: 15739458.6
(22) Date of filing: 08.07.2015
(51) Int. Cl.: A61B 17/15, A61B 34/10, A61F 2/46

(54) **PATIENT-SPECIFIC KNEE ALIGNMENT GUIDE**
PATIENTENSPEZIFISCHE KNIEAUSRICHTUNGSFÜHRUNG
GUIDE D'ALIGNEMENT DE GENOU SPÉCIFIQUE À UN PATIENT

(30) Priority: 09.07.2014 US 201414327234
(43) Date of publication of application: 17.05.2017
(73) Proprietor: Biomet Manufacturing, LLC, Warsaw, IN 46582 (US)
(72) Inventor: METZGER, Robert, Wakarusa, IN 46573 (US); BEREND, Keith R., New Albany, OH 43054 (US); BEREND, Michael E., Indianapolis, IN 46202 (US); LOMBARDI, Adolph V., New Albany, OH 46054 (US); PERRY, Lance D., Warsaw, IN 46582 (US); SCHOENEFELD, Ryan J., Fort Wayne, IN 46804 (US); WILLIAMSON, Daniel E., Warsaw, IN 46580 (US); STEGER, Shon D., Leesburg, IN 46580 (US)
(74) Representative: Mays, Julie
(86) International application number: PCT/US2015/039561
(87) International publication number: WO 2016/007631

(56) References cited:
- WO-A1-2011/063231
- US-A1- 2011 015 636
- US-A1- 2011 035 012
- US-A1- 2012 143 267

## Description

### INTRODUCTION

Proper alignment of prosthetic components in knee arthroscopy is an important factor in the longevity and function of the implant. Misalignment can cause increased wear of the implant, patient discomfort, and functional limitation. Document WO 2011/063231 A1 describes a system comprising a guide, the guide having a guiding aperture that when the guide is placed on bone is directed to a planned location relative to soft tissue, an instrument configured to be guided by the guiding aperture and form a first hole to the planned location into the bone and a syringe having a member configured to be placed in the formed first hole.

Although various methods and devices are known for addressing the above problems, patient specific alignment methods and alignment guides are still desirable.

### SUMMARY

The present invention is defined in claim 1 and may be used in a method of preparing a joint for a prosthesis in a patient. In one aspect, the method includes obtaining scan data associated with the joint of the patient, preparing a three-dimensional image of the joint based on the scan data, preparing an interactive initial surgical plan based on the scan data, sending the surgical plan to a surgeon, receiving a finalized surgical plan from surgeon, and preparing an image of a patient-specific alignment guide.

In another aspect, the method includes securing a patient-specific alignment guide to a joint surface of the patient, attaching a guide element through the alignment guide to the joint surface, removing the alignment guide without removing the guide element, and resecting the joint surface using the guide element.

The present invention may also be used in a method of preparing a knee joint for a prosthesis in a patient. The method includes locking a patient-specific femoral alignment guide onto a femoral joint surface of the patient, inserting at least one first guide element through the femoral alignment guide into the anterior or the anterior-medial side of the femoral joint surface, and drilling resection-locating apertures in the distal side of femoral joint surface. The method further includes removing the femoral alignment guide without removing the first guide element, supporting a femoral resection device on the first guide element, and resecting the femoral joint surface.

The present invention provides an orthopedic device for preparing a knee joint for a prosthesis in a patient. The orthopedic device includes a femoral alignment guide having a patient-specific three-dimensional curved inner surface. The curved inner surface is preoperatively configured from medical image scans of the knee joint of the patient to nestingly conform and mate and match only in one position to a corresponding three-dimensional femoral surface of a joint surface of the patient. The femoral alignment guide has a first guiding aperture corresponding to a distal portion of the femoral surface and a second guiding aperture corresponding to an anterior portion of the femoral surface.

A method of preparing a knee joint for a prosthesis in a patient is disclosed. The method includes mating a patient-specific three-dimensional curved inner surface of a femoral alignment guide onto a corresponding three-dimensional femoral joint surface of the patient, the patient-specific three-dimensional curved inner surface preoperatively configured from medical scans of the knee joint of the patient, drilling a first hole into an anterior portion of the femoral joint surface through a corresponding first guiding aperture of the femoral alignment guide and drilling a second hole into an anterior portion of the femoral joint surface through a corresponding second guiding aperture of the femoral alignment guide. In the method, the second guiding aperture is asymmetrically located relative to the first guiding aperture on the femoral alignment guide. The method can further include mating a portion of the inner surface of the femoral alignment guide to articular cartilage covering the femoral joint surface. The method can further include mating a portion of the inner surface of the femoral alignment guide to a bone portion underlying articular cartilage of the femoral joint surface. The method can further include inserting first and second guiding pins through the corresponding first and second guiding apertures and first and second holes. The method can further include removing the femoral alignment guide without removing the first and second guiding pins. The method can further include sliding the femoral alignment guide through open portions of corresponding perimeters of the first and second guiding apertures. The method can further include supporting a cutting block on the first and second guiding pins. The method can further include guiding a patient-specific femoral resection through the cutting block, the patient-specific resection determined by preoperative configuring the first and second guiding apertures on the femoral alignment guide to correspond to the patient-specific resection.

A method of preparing a knee joint for a prosthesis in a patient may include mating a patient-specific three-dimensional curved inner surface of a femoral alignment guide onto a corresponding three-dimensional femoral joint surface of a patient, the patient-specific three-dimensional curved inner surface preoperatively configured from medical scans of the knee joint of the patient, drilling first and second holes into an anterior portion of the femoral joint surface through corresponding first and second guiding apertures of the femoral alignment guide, wherein the first and second guiding apertures are preoperative configured for locating a femoral resection of the patient according to the medical scans of the patient and a preoperative surgical plan for the patient, and inserting first and second guiding pins through the corresponding first and second guiding apertures and the first and second holes. The method may further include removing the femoral alignment guide without removing the first and second guiding pins. The method may further include sliding the femoral alignment guide through open portions of corresponding perimeters of the first and second guiding apertures.. The method may further include supporting a cutting block on the first and second guiding pins. The method may further include guiding a patient-specific femoral resection through the cutting block. The method may further include mating a portion of the inner surface of the femoral alignment guide to at least one of articular cartilage and underlying bone of the femoral joint surface.

A method of preparing a knee joint for a prosthesis in a patient can include mating a patient-specific three-dimensional curved inner surface of a tibial alignment guide onto a corresponding three-dimensional tibial joint surface of the patient, the patient-specific three-dimensional curved inner surface preoperatively configured from medical scans of the knee joint of the patient, and wrapping a portion of the tibial alignment guide around an anterior-medial edge of the tibial joint surface. The method may further include drilling a first tibial guiding hole into an anterior portion of the tibial joint surface through a first corresponding anterior aperture of the tibial alignment guide. The method may further include drilling a second tibial guiding hole into an anterior portion of the tibial joint surface through a second corresponding anterior aperture of the tibial alignment guide. The method may further include inserting first and second guiding pins through the corresponding first and second anterior apertures and into the corresponding first and second tibial guiding holes. The method may further include removing the tibial alignment guide without removing the first and second guiding pins by sliding the tibial alignment guide through open portions of corresponding perimeters of the first and second anterior apertures of the tibial alignment guide.

Further areas of applicability of the present invention will become apparent from the description provided hereinafter. It should be understood that the description and specific examples are intended for purposes of illustration only and are not intended to limit the scope of the invention.

### BRIEF DESCRIPTION OF THE DRAWINGS

The present invention will become more fully understood from the detailed description and the accompanying drawings, wherein:
FIG. 1 is a flowchart of an exemplary method of preparing patient specific alignment guides according to the present teachings;
FIG. 2 is a flowchart of an alignment method according to the present teachings;
FIG. 3 is a view illustrating the mechanical axis in a patient's anatomic image;
FIG. 4 is a view illustrating the transepicondylar and cylindrical axes in a patient's anatomic image;
FIG. 5 is a view illustrating the mechanical and anatomic axes in a patient's femoral image;
FIG. 6 is a flowchart of an exemplary method of using patient specific alignment guides according to the present teachings;
FIG. 7 is an exemplary image of a patient's anatomy with implants shown, as viewed in interactive software according to the present teachings;
FIG. 8 is a perspective view of an exemplary femoral alignment guide according to the present teachings, shown next to a corresponding anatomic femur;
FIGS. 9A and 9B are perspective views of the femoral alignment guide of FIG. 8 shown mounted on the femur;
FIGS. 10A and 10B are perspective views of the femoral alignment guide of FIG. 8 shown with spring pins securing the alignment guide to the femur;
FIG. 11A is a perspective view of the femoral alignment guide of FIG. 8 shown with a drill guide;
FIG. 11B is a perspective view of the femoral alignment guide of FIG. 11A shown with two guide pins drilled through the drill guide;
FIG. 11C is perspective view of the femoral alignment guide of FIG. 11B showing the removal of the drill guide;
FIG. 12A is a perspective view of the femoral alignment guide of FIG. 11C shown after the removal of the drill guide;
FIG. 12B is a perspective view of the femoral alignment guide of FIG. 12A shown after the removal of the spring pins;
FIG. 13A is a perspective view of FIG. 12B illustrating the guide pins after the removal of the femoral alignment guide;
FIG. 13B illustrates a detail of the femoral alignment guide of FIG. 12B;
FIG. 14A is a perspective view of a distal femoral cutting block shown over two pins on a patient's femur, according to the present teachings;
FIG. 14B is a perspective view of a distal femoral cutting block shown over two guide pins on a patient's femur, according to the present teachings;
FIG. 15A is a perspective view of an exemplary 4-in-1 cutting block positioned on the femur with reference to holes corresponding to the spring pins;
FIG. 15B a perspective view of the cutting block of FIG. 15A shown with a cutting blade;
FIG. 16A is a perspective view of a tibial alignment guide according to the present teachings, shown mounted on the tibia;
FIG. 16B is a perspective view of the tibial alignment guide of FIG. 16A shown with a drill guide;
FIG. 16C is a perspective view of FIG. 16B illustrating the guide pins after the removal of the tibial alignment guide;
FIG. 16D is a perspective view of FIG. 16C illustrating a tibial cutting guide mounted on the guide pins;
Fig. 17 is a perspective environmental view of a distal femur with a guide positioned thereon and an instrument forming a bore in the femur;
Fig. 17A is a perspective view of the guide of Fig. 17; and
Fig. 18 is an environmental view of a distal femur with a guide positioned thereon and an instrument position in a bore through the guide.

### DESCRIPTION OF VARIOUS ASPECTS

The following description is merely exemplary in nature and is in no way intended to limit the scope of the present teachings, applications, or uses. For example, although the present teachings are illustrated for alignment guides in knee surgery, the present teachings can be used for other guides, templates, jigs, drills, rasps or other instruments used in various orthopedic procedures.

The present teachings provide a method for preparing patient-specific alignment guides for use in orthopedic surgery for a joint, such as, for example, the knee joint. Conventional, not patient-specific, prosthesis components available in different sizes can be used with the alignment guides, although patient-specific femoral and tibial prosthesis components prepared with computer-assisted image methods can also be used. Computer modeling for obtaining three dimensional images of the patient's anatomy, such as a patient's joint, for example, the patient-specific prosthesis components, when used, and the alignment guides and templates can be provided by various CAD programs and/or software available from various vendors or developers, such as, for example, from Materialise USA, Ann Arbor, Michigan.

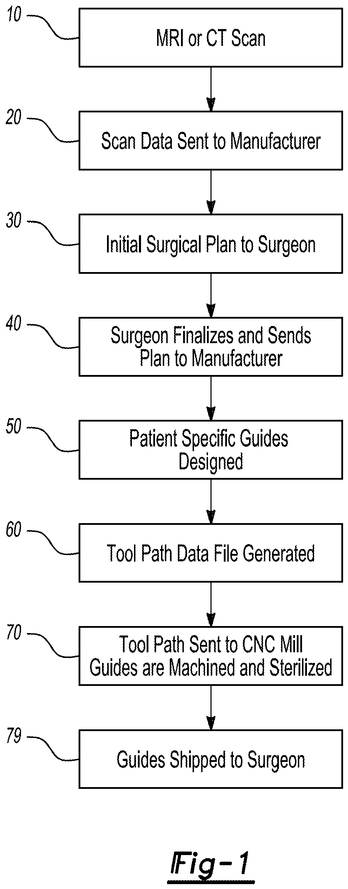

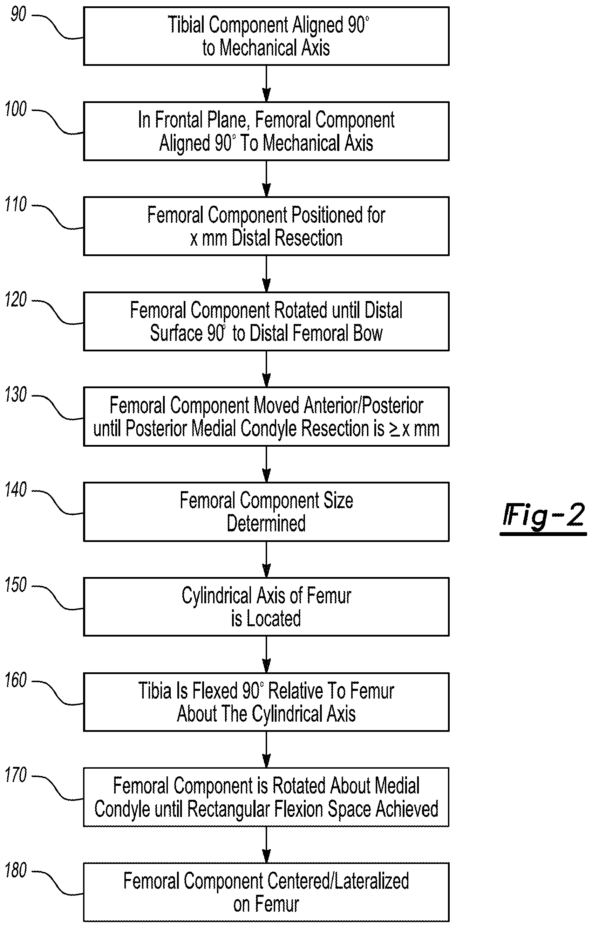

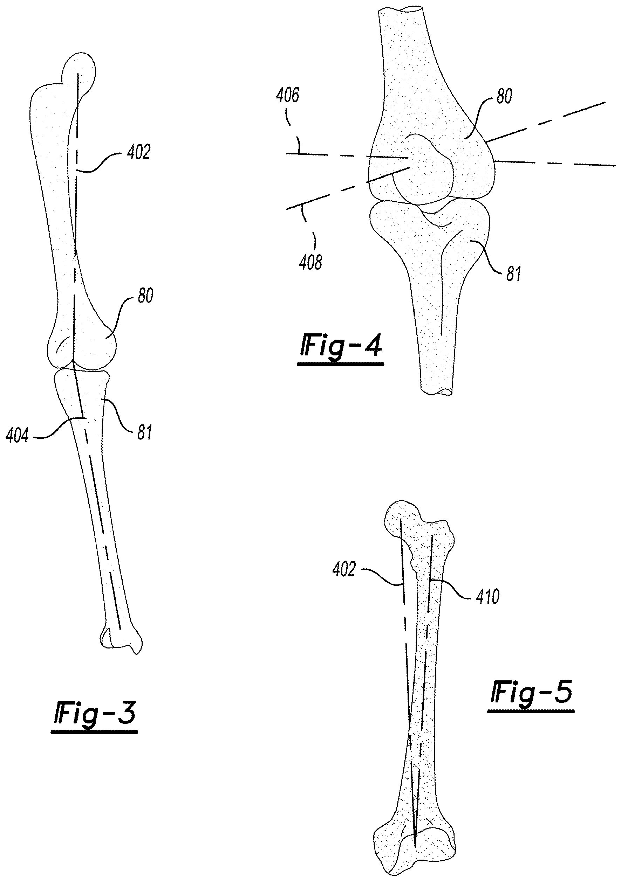

Referring to FIG. 1, an MRI scan or a series of CT scans of the entire leg of the joint to be reconstructed, including hip and ankle, as shown in FIG. 3, can be performed at a medical facility or doctor's office, at aspect 10. In some cases, the scan may be performed with the patient wearing an unloader brace to stress the ligaments. The scan data obtained can be sent to a manufacturer, at aspect 20. The scan data can be used to construct a three-dimensional image of the joint and provide an initial implant fitting and alignment in a computer file form or other computer representation. The initial implant fitting and alignment can be obtained using an alignment method, such as the alignment method illustrated in FIG. 2 and described below. Other alignment methods can also be used, such as alignment protocols used by individual surgeons.

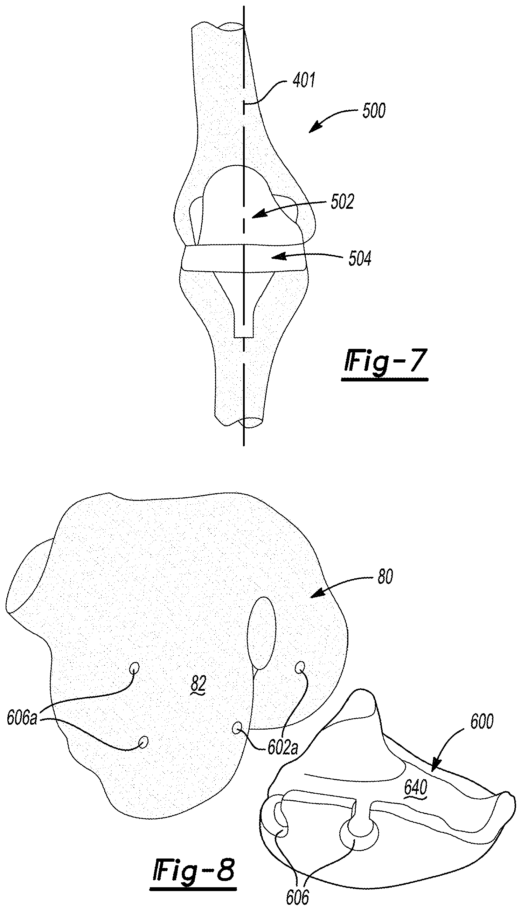

The outcome of the initial fitting is an initial surgical plan that can be printed or provided in electronic form with corresponding viewing software. The initial surgical plan can be surgeon-specific, when using surgeon-specific alignment protocols. The initial surgical plan, in a computer file form associated with interactive software, can be sent to the surgeon, or other medical practitioner, for review, at 30. The surgeon can incrementally manipulate the position of images of implant components 502, 504 in an interactive image form 500 of the joint, as illustrated in FIG. 7. After the surgeon modifies and/or approves the surgical plan, the surgeon can send the final, approved plan to the manufacturer, at 40.

Various methods of sending the initial and final surgeon-approved surgical plans can be used. The surgical plans can be, for example, transferred to an electronic storage medium, such as CD, DVD, flash memory, which can then be mailed using regular posting methods. Alternatively, the surgical plan can be e-mailed in electronic form or transmitted through the internet or other web-based service, without the use of a storage medium.

After the surgical plan is approved by the surgeon, patient-specific alignment guides for the femur and tibia can be developed using a CAD program or other imaging software, such as the software provided by Materialise, for example, according to the surgical plan, at 50. Computer instructions of tool paths for machining the patient-specific alignment guides can be generated and stored in a tool path data file, at 60. The tool path can be provided as input to a CNC mill or other automated machining system, and the alignment guides can be machined from polymer, ceramic, metal or other suitable material, and sterilized, at 70. The sterilized alignment guides can be shipped to the surgeon or medical facility, at aspect 79 for use during the surgical procedure.

Referring to FIG. 2, an exemplary method for providing the initial implant fitting and alignment is illustrated. The method can be modified or completely replaced according to a surgeon-specific alignment protocol. After the scan data is converted to three dimensional images of the patient anatomy from hip to ankle, images of the tibial and femoral components can be manipulated for obtaining patient-specific alignment by making use of the femoral and tibial mechanical axes 402, 404, illustrated in FIG. 3, and the transepicondylar and cylindrical axes 406, 408, illustrated in FIG. 4. Images of the knee joint anatomy can include images of the joint surfaces of the distal femur and proximal tibial with or without the associated soft tissues, such as articular cartilage, on the respective bone surfaces.

Generally, the femoral mechanical axis is defined as the line joining the center of the femoral head and the center of the intercondylar notch. The femoral anatomic axis is defined as the line along the center of the femoral shaft. The tibial mechanical axis is the line joining the center of the tibial plateau to the center of the tibial plafond or the center of the distal end of the tibia. The tibial anatomic axis is the line along the center of the tibial shaft. The transepicondylar axis is the line connecting the most prominent points of the epicondyles. The cylindrical axis is the line connecting the centers of the condyles when the condyles are approximated by coaxial cylinders. A detailed discussion of the various joint-related axes and the relation of the transepicondylar axis 406 and cylindrical axis 408 is provided in Eckhoff et al, Three-Dimensional Mechanics, Kinematics, and Morphology of the Knee Viewed in Virtual Reality, J Bone Joint Surg Am. 87:71-80, 2005, which is incorporated herein by reference.

The relation of the femoral mechanical axis 402 to the anatomic axis 410 for the femur is illustrated in FIG. 5. The femoral and tibial mechanical axes 402, 404 may or may not coincide, as illustrated in FIG. 3. In the following discussion, reference is made to a single mechanical axis 401 encompassing the femoral and tibial mechanical axes 402, 404. The alignment procedure illustrated in FIG. 2 makes use of the mechanical, anatomic, transepicondylar and cylindrical axes in various degrees. The present teachings, however, are not limited to this alignment procedure. Multiple alignment procedures can be provided to accommodate the experience and preference of individual surgeons. For example, the alignment procedure can be based on the anatomic and mechanical axes, or can be substantially based on the cylindrical axis. Further, the alignment procedure can be deformity-specific, such that is adapted, for example, to a valgus or varus deformity.

With continued reference to FIG. 2-5 and 7, in the image space, the tibial component 504 can be aligned 90° to the mechanical axis 401, at aspect 90. In the frontal plane, the femoral component 502 can be aligned 90° to the mechanical axis 401, at aspect 100. The femoral component 502 can be positioned for "x" mm distal resection, at 110, where "x" can be about 9 mm or as other measurement as indicated for a specific patient. The femoral component 502 can be rotated until its distal surfaces are at 90° to the distal femoral bow (component flexion/extension), at 120. The femoral component 502 can be moved anteriorly/posteriorly until the posterior medial condyle resection is greater or equal to "x" mm, at aspect 130.

The femoral component size can be determined by observing the anterior resection relative to anterior cortex, at 140. If the femoral size is adjusted, the new size can be positioned at the same location relative to the distal and posterior cut planes.

The cylindrical axis 408 of the femur can be located, at aspect 150. The tibia can be flexed 90° relative to the femur about the cylindrical axis 408, at aspect 160. The femoral component 502 can be rotated about the medial condyle until a rectangular flexion space is achieved, at aspect 170. Alternatively, the rotation can be relative to the transepicondylar axis, anterior/posterior axis, and posterior condylar axis, or a combination of all four axes. The femoral component 502 can be centered or lateralized on the femur, at aspect 180. The location for various distal holes for locating the femoral resection block can be also determined.

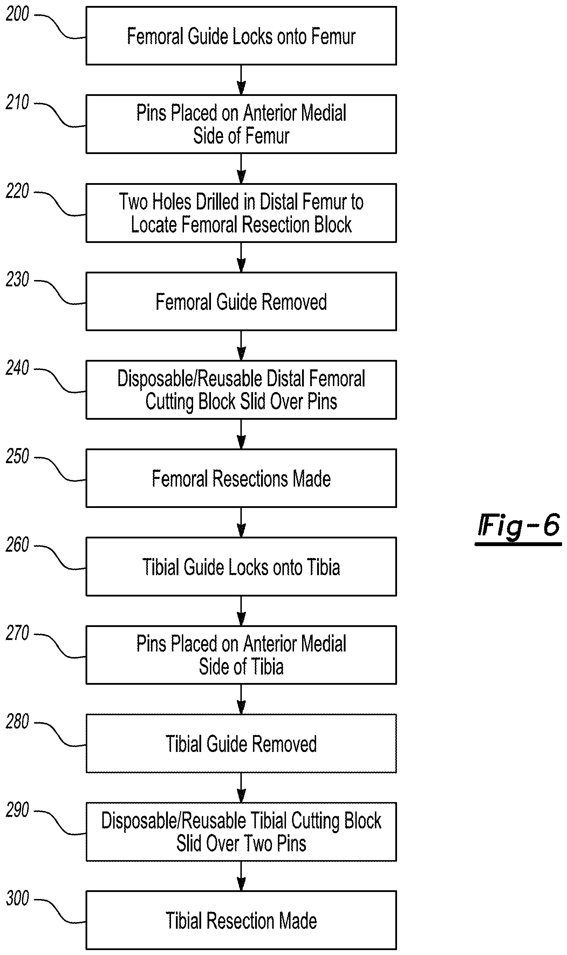

Referring to FIGS. 6, and 8-15B, an exemplary alignment guide 600 and method of use is illustrated in connection with the patient's femur 80. Reference numbers 200-250 relate to aspects of the method of FIG. 6 and are described in connection with the instruments shown in FIGS. 8-15B for the femur 80.

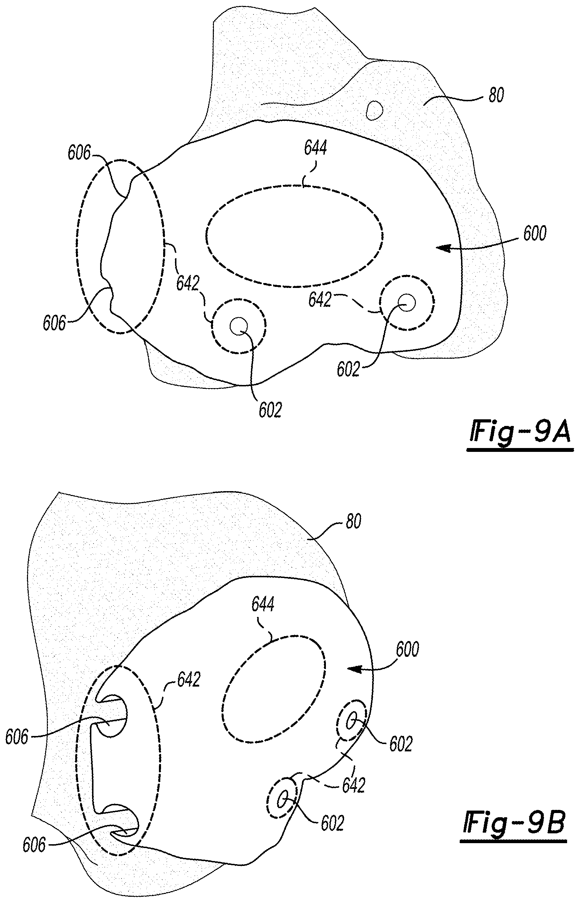

The alignment guide 600 includes an inner guide surface 640 designed to closely conform, mate and match the femoral joint surface 82 of the patient in three-dimensional space such that the alignment guide 600 and the femoral joint surface are in a nesting relationship to one another. Accordingly, the alignment guide 600 can conform, mate and snap on or "lock" onto the distal surface of the femur 80 in a unique position determined in the final surgical plan, at 200. The alignment guide 600 can have variable thickness. In general, the alignment guide 600 can be made as thin as possible while maintaining structural stiffness. For example, certain areas around and adjacent various securing or guiding apertures 602, 606 can be thickened to provide structural support for guiding a drill or for holding a drill guide or supporting other tools or devices. Exemplary thickened areas 642 are indicated with dotted lines in FIGS. 9A and 9B. Other areas can be cut out for viewing the underlying bone or cartilage of femoral joint surface 82. Viewing areas 644 are indicated with dotted lines in FIGS. 9A and 9B.

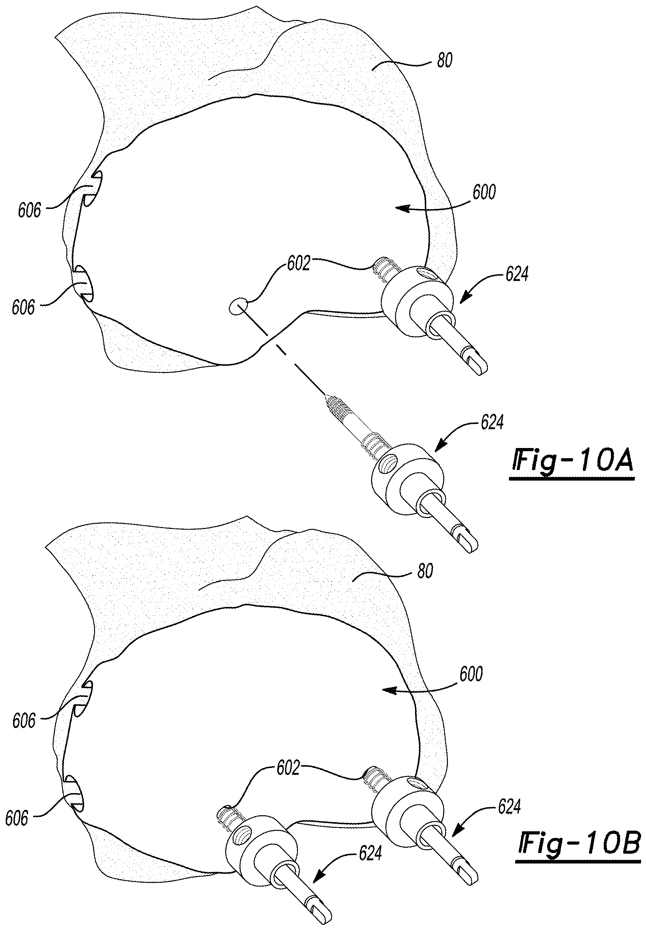

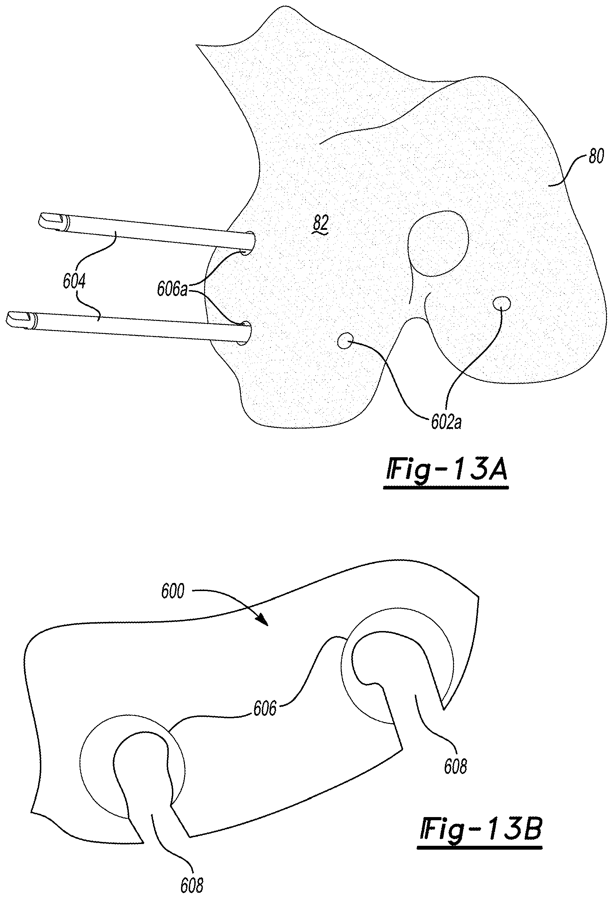

Referring to FIGS. 10A and 10B, the alignment guide 600 can be secured to the femoral joint surface 82 with fixation members or fasteners 624, such as, for example, spring pins, or other securing fasteners that are received through distal apertures 602 of the alignment guide 600. Locating holes 602a corresponding to the apertures 602 of the alignment guide 600 can be drilled in the distal femur 80 to locate a femoral resection block or other cutting device 620, such as a 4-in-1 cutting block, at 220. The alignment guide 600 can also include guiding apertures 606. Guiding apertures 606 are shown in the anterior-medial side relative to the femur 80, but can also be made in the anterior side of the femur 80 or in other locations and orientations. The guiding apertures 606 can be counter-bored and have a partially open portion 608 in their perimeter for sliding the alignment guide off pins or other fasteners without removing such fasteners, as shown in FIG. 13A and discussed below.

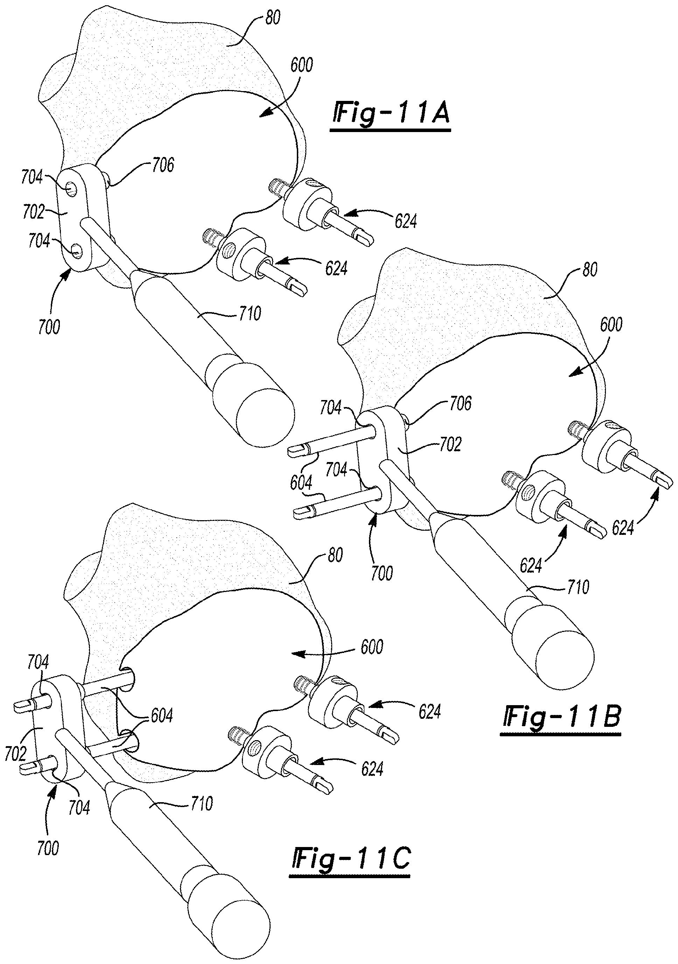

Referring to FIGS. 11A and 11B, a drill guide 700 can be placed in alignment with the guiding apertures 606. The drill guide 700 can include a body 702 having guiding bores 704 corresponding to the guiding apertures 606. The guiding bores 704 can have portions 706 that extend beyond the body 702 and into the guiding apertures 606 for facilitating alignment. The drill guide 700 can also include a handle 710 extending sideways from the body 702 and clear from the drilling path.

Referring to FIG. 11C, guide elements 604, such as pins or other fasteners, for example, can be drilled through the guiding bores 704 of the drill guide 700 on the anterior or anterior-medial side of the femur 80, at aspect 210 of the method of FIG. 6. The guide elements 604 can be parallel or at other angles relative to another. The guide elements 604 can define a plane that is parallel to a distal resection plane for the femur.

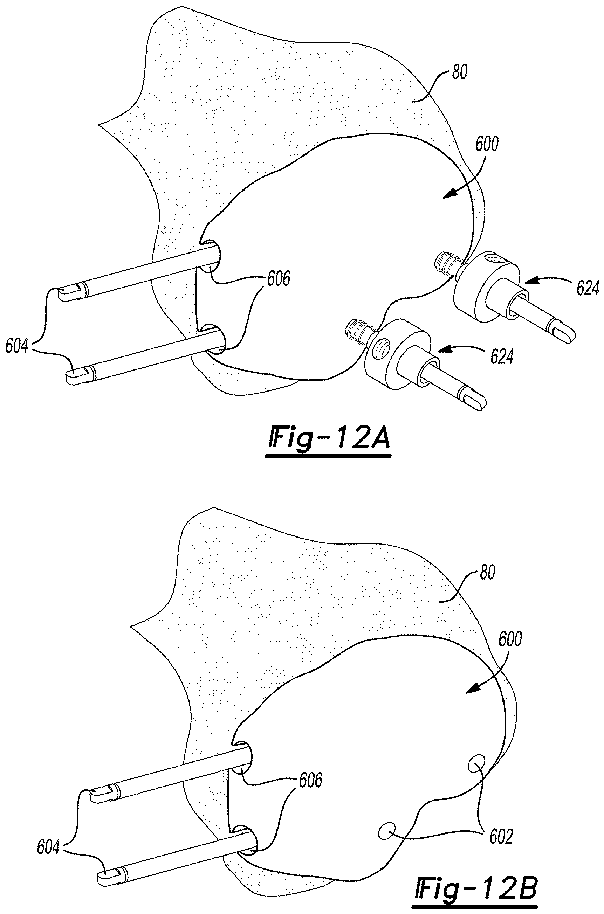

Referring to FIG. 12A, the drill guide 700 can be removed. Referring to FIGS. 12B-13B, the fasteners 624 can be removed, and the alignment guide 600 can be removed from the femur 80 by sliding the alignment guide 600 off the guide elements 604 through the open portions 608 of the guiding apertures 606 without removing the guide elements 604 at the anterior/medial corner of the knee, at aspect 230 of FIG. 6.

The guide elements 604 can be used to prepare the joint surfaces for the prosthesis by mounting cutting guides/blocks for resecting the joint surface. Alternatively, a robotic arm or other automated, guided or computer controlled device that can guide the resections based on the pre-operative surgical plan can be mounted on the guide elements 604 and assist the surgeon in preparing the joint surface for the prosthesis.

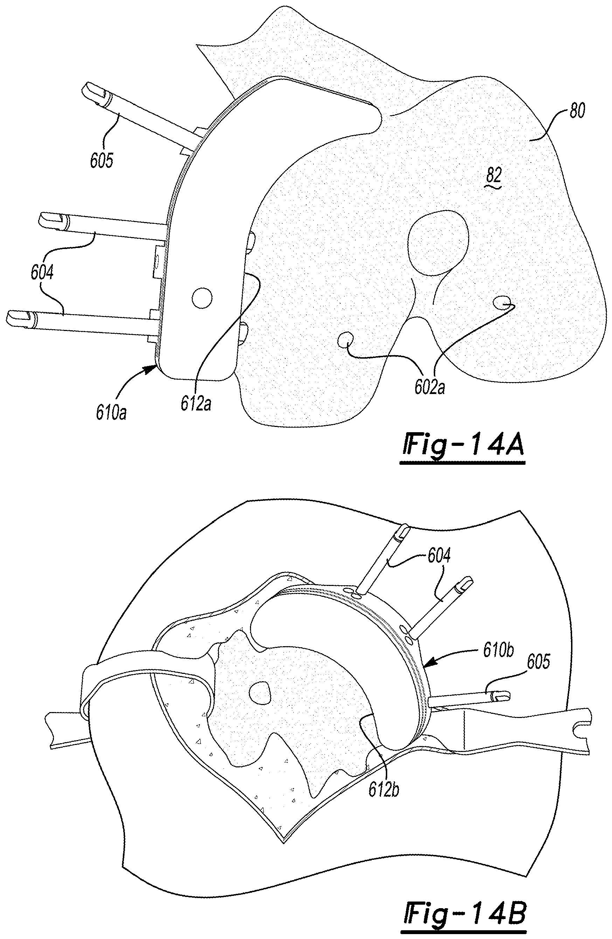

Referring to FIGS. 14A and 14B, exemplary distal cutting blocks 610a, 610b that can be mounted over the guide element 604 for making the distal resection, at aspect 640 of FIG. 6, are illustrated. A third fixation element 605, obliquely oriented relative to the guide elements 604 can also be used. The distal cutting blocks 610a, 610b can have an inner surface 612a, 612b that generally follows the shape of the femur 80 to a lesser or greater degree. The distal cutting blocks 610a, 610b can be disposable or re-usable.

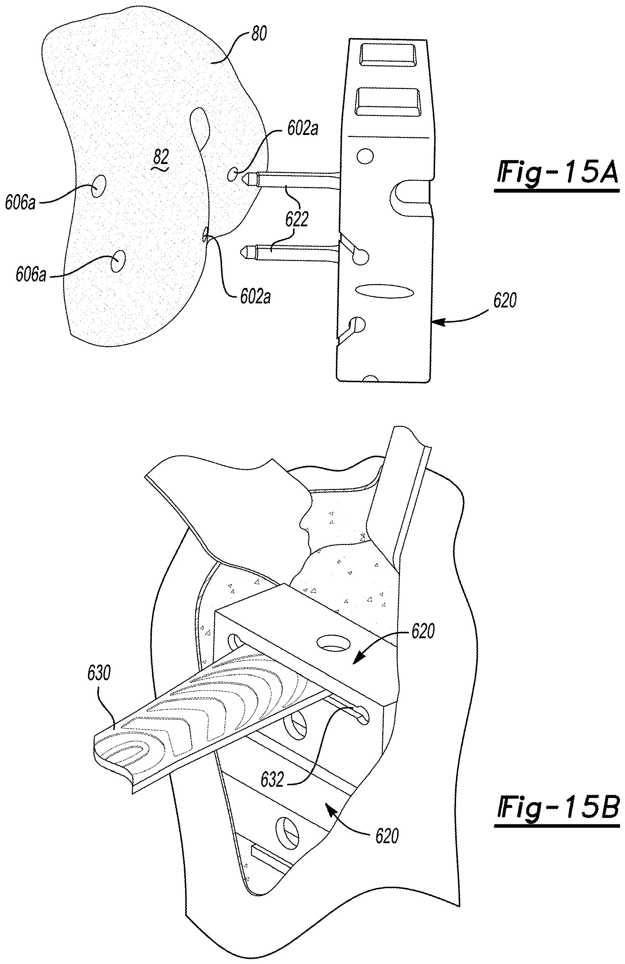

Referring to FIGS. 15A and 15B, after the distal resections are made with the distal cutting block 610a or 610b, the femoral resection block 620 can be mounted with pegs or other supporting elements 622 into the holes 602a corresponding to the fasteners 624. The femoral resections can be made using, for example, a cutting blade 630 through slots 632 of the femoral resection block 620, at aspect 250 of FIG. 6.

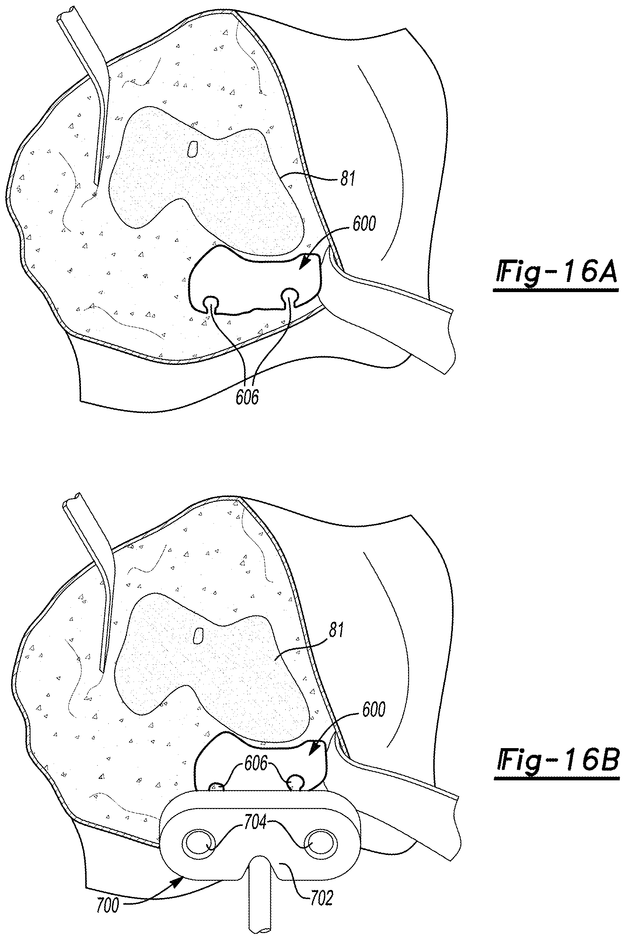

Referring to FIGS. 6 and 16A-D, an exemplary alignment guide 600 is illustrated in connection with the patient's tibia 81. Reference numbers 260-300 relate to aspects of the method of FIG. 6 and are described in connection with the instruments shown in FIGS. 16A-16D for the tibia.

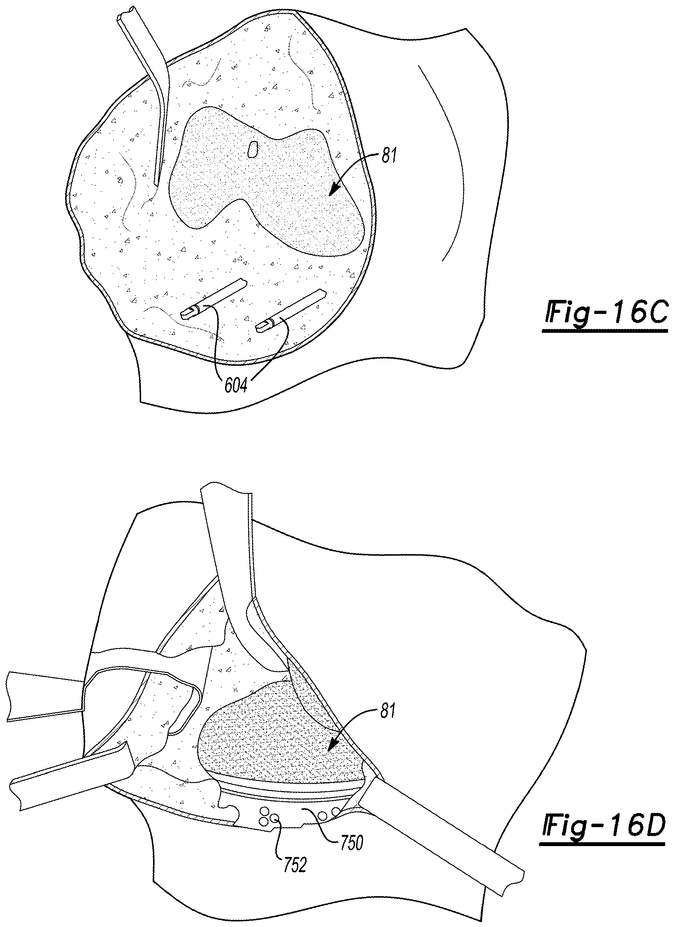

The alignment guide 600 can conform, nestingly mate in three-dimensional space and snap on or "lock" by design onto the tibia 81 in a unique position, at aspect 260 of FIG. 6. The alignment guide 600 can wrap around the anterior-medial edge of the tibia 81, as shown in FIG. 16A. The drill guide 700 can be aligned with the counter-bored guiding apertures 606 of the alignment guide 600, as shown in FIG. 16B. Two or more guide elements 604 can be placed on the anterior medial side of the tibia, at aspect 270 of FIG. 6. An additional fixation element can also be used for additional securing for the alignment guide 600. The drill guide 700 and the alignment guide 600 can be removed, leaving behind the guide elements 604 attached, at aspect 280 of FIG. 6, and as shown in FIG. 16C. A disposable or reusable tibial cutting block 750 can be slid over the guide elements 604, at aspect 290 of FIG. 6, and as shown in FIG. 16D. The tibial cutting block 750 can include a series of holes 752, allowing the cutting block 750 to be translated proximally or distally to adjust the level of the distal resection. The tibial resection can be made, at 300.

The present teachings provide patient-specific alignment guides that can be used for alignment in orthopedic surgery. Each alignment guide includes an inner surface that nestingly mates and conforms in three-dimensional space with a corresponding joint surface of a specific patient. The alignment guides can be used for locating guide elements on the joint surface. After the alignment guides are removed, cutting guides or other cutting devices, including automated or robotic devices, can be mounted on the guide elements for making various resection cuts. Because the alignment guides are not used for cutting, the alignment guides do not require substantive thickness to extend anteriorly, and consequently have a lower profile, and less weight. Additionally, because the alignment guides are removed before cutting, the present teachings provide increased ability to visualize the cuts and the cutting process.

### Subchondral Access

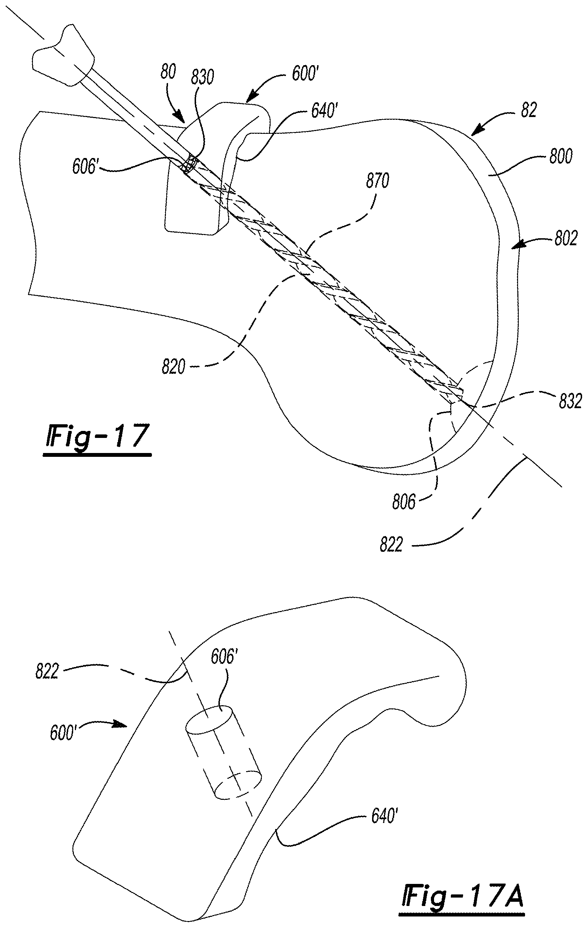

According to various embodiments, the guide 600 or any appropriate guide member, including the guide 600' illustrated in Fig. 17, can be positioned relative to a bone, such as the femur 80, for performing a procedure relative to the bone. For example, as illustrated in Fig. 17, the guide 600' can be positioned relative to the femur 80 to guide a drilling member, such as a drill bit or pin 820, to obtain access to an interior portion of the femur 80 that is beneath a surface of the femur. Once access is obtained to a portion of the femur, such as below the surface of a femur, a procedure can be performed on the femur for various purposes. For example, the procedure can include a tumor resection, a bone filling, and/or a bone replacement.

According to various procedures, a portion of bone that is beneath a condyle, also referred to as subchondral, can be repaired or replaced with a bone void filling material, such as in a procedure generally disclosed using Subchondroplasty® instruments and/or access devices that are provided by Knee Creations LLC, having a place of business in Westchester, Pennsylvania, USA and/or Subchondroplasty Orthopedics LLC having a place of business in Westchester, Pennsylvania, USA. The Subchondroplasty® instruments are known to assist in obtaining access to an area of a bone that is beneath a condyle to perform a procedure interior to the bone to assist in maintaining the condyle region, including a condylar cartilage. For example, a subchondular bone can be reinforced or strengthened with a selected material to strengthen the bone and to reinforce and strengthen support of the condylar cartilage. Appropriate materials can be biomimetic bone substitute material that can be used to strengthen and/or cause replacement of subchondular bone.

Regions that may need to be strengthened include bone marrow lesions that form and may weaken support of the condylar cartilage. Accordingly, the appropriate bone substitute materials can be placed in or around the bone marrow lesions to cause regeneration of the bone and strengthening thereof. Appropriate bone substitute materials can include Accufill® bone void filler that includes injectable calcium phosphate, provided by Knee Creations, LLC having a place of business in New York, New York, USA. According to various embodiments, bone void filler materials, including calcium phosphate, can be placed in a bone defect to cause regrowth and/or remineralization of a selected bone region.

It is also understood that the guide 600 can be used to place an instrument, such as the guide elements 604, through one or more guiding apertures 606 to position the instrument at an appropriate and selected location within the bone. For example, as discussed above, the guide 600 can be designed to include the inner guide surface 640 that closely conforms or matches a femoral joint surface 82. The inner guide surface 640 is generally or specifically a three-dimensional curved inner surface that is preoperatively configured from medical image scans of the patient, such as a knee joint of the patient, to nestingly conform and mate and match only in one position to a corresponding three-dimensional femoral surface of the patient. The guiding apertures 606, therefore, are positioned at a selected, known, and specific location relative to the femur 80. The design of the inner guide surface 640 and the placement of the guiding aperture 606 may assist in a guiding and insuring guiding of any appropriate instrument relative to an interior portion of the bone, including the femur 80. The apertures 606' can define a patient specific axis, as discussed herein, to guide an instrument to form a patient specific hole or bore and to a patient specific location. Thus, any appropriate procedure can be performed at an interior of the bone. Appropriate procedures can further include removing a tumor within the bone, removing necrotic tissue within the bone, positioning a bone-growing implant, positioning a supporting device within the bone, or other appropriate procedure.

It is understood that the guide 600, or any appropriate guide, can be designed to mate with a surface of any appropriate bone portion including a distal femoral bone portion surface, a distal anterior femoral bone surface, a vertebral bone surface, a proximal tibial bone surface, or any other appropriate bone surface. Further, a procedure can be performed with the guide 600, according to various embodiments to assist in fixation of bone, implanting an implant transverse through a fracture, tumor removal, bone surface supporting implant or device, or other appropriate procedure.

With reference to FIG. 1, scan data can be generated or obtained at aspect 10. The scan data can include various image data of a patient including MRI scan data and CT scan data. The scan data can be used to generate an initial surgical plan at aspect 30 which is finalized at aspect 40 to design patient-specific guides at aspect 50. The patient specific guides are then made, including being machined, at aspect 70 and are then shipped to a surgeon at aspect 79. The plan that is finalized by the surgeon at aspect 40 can be based upon various diagnoses of a patient. For example, a diagnosis of a bone loss, tumor, fracture, and the like can be made based upon the scan data of the patient. The finalized plan can include placement and/or orientation of a guiding aperture 606' in the guide 600' such that instruments are guided to selected and known diagnosed regions of the patient. It will be understood that although the following discussion relates generally to a subchondrol weakening or lesion relative to a distal femur in a knee joint, any diagnosis can be made and that the guide 600' can be designed, according to method illustrated in FIG. 1, to allow for positioning of an instrument relative to the diagnosed region.

Turning reference to FIGS. 17 and 17A, the femur 80 is schematically illustrated. The femur 80 can be illustrated as image data on a display device, such as a computer display device. The FIG. 17 illustrating the femur 80 can represent a natural femur and/or image data or a conversion of image data into a three-dimensional display for viewing by a user and/or operator, including a surgeon. The femur 80 can include the condylar cartilage 800 that can extend over at least a portion of the femur 80 including a condylar region 802 that can define at least a portion of the femoral joint surface 82. Other portions of the femur 80 may not include cartilage, such as medial and/or anterior regions of the femur 80. As illustrated and discussed above, the femoral joint surface 82 can form at least a portion of the knee joint where the femur can articulate with a proximal portion of the tibia 81. Near the condyle 82 and generally below the condylar cartilage 800, a bone lesion and/or weakened bone region 806 can be viewed (i.e. seen in the scan data of the patient) and/or diagnosed. The weakened bone region 806 can be diagnosed or viewed based upon generally known techniques, including viewing the scans, e.g. MRI and CT scans, of the patient and/or a conversion or reconstruction of the scanned data by one skilled in the art. It will be understood that the weakened bone region 806 can be representative of any appropriate diagnosis, including diagnosis of a tumor, fracture, bone marrow lesions, or other bone defect or area for performing a procedure.

Once the weakened bone region 806 is diagnosed in the femur 800, the method illustrated in FIG. 1 can be used to design a patient-specific guide at aspect 50, machine and sterilize the patient-specific guide at aspect 70, and ship the guide to the surgeon at aspect 79. The patient-specific guide 600' can include an inner patient specific surface 640' to contact a selected portion of a surface of the femur 80. It is understood that the guide 600' can be designed to contact a medial, lateral, anterior, anterior medial, anterior lateral, or any appropriate portion of the bone. The patient specific guide 600' and the inner surface 640' can be similar to the guide 600 and inner surface 640 discussed above in that the guide can contact, nest, and/or lock to a specific portion of the anatomy to align the patient specific guiding aperture 606' relative to a specific region, such as the weakened region 806.

As exemplarily illustrated in FIG. 17, an anterior-medial positioning of a patient-specific guide 600' is illustrated. The guide 600' may include one or more of the guide apertures 606' that can be formed through the guide 600'. An instrument, including the guide member 604, the drill 820, or other appropriate member can be positioned through the aperture 606' to form a portal or bore 870 into the femur 80. The bore 870 may be a blind bore that terminates away from the cartilage 800 such that a port or hole is not formed into or through the cartilage 800. Thus, the bore 870 will generally extend only through the bone material and the bore forming instrument need not contact or engage the cartilage 800.

The drill bit 820 can be passed through the guide 600', such as through the guide aperture 606', to engage the femur 800 and drill to the weakened bone region 806. The positioning of the guide 600' and the guiding aperture 606' can be such that the drill 820 will proceed along a patient specific axis 822 through the femur 800 and to the weakened bone region 806. Based upon the scan data from aspect 10 of FIG. 1 and the plan at aspect 40 a patient specific mark can also be placed on the drill bit 820, including a visible demarcation 830, such that the surgeon knows when the drill bit 820, including a tip 832, has moved an appropriate amount into the femur 80. The appropriate amount can include the tip 832 being positioned at the weakened bone region 806. The surgeon may then stop progressing the drill 820 into the femur 80 and perform a procedure through the bore 870 at a patient specific depth.

Once the drill bit 820 has been positioned such that at least the tip 832 has reached the weakened bond region 806, a procedure can occur relative to the femur 80 and the weakened bone region 806. According to various embodiments, a material may be delivered to the weakened bone region 806, such as a bone void filling material, other pharmaceutical, steroid, demineralized bone, or other appropriate material. According to various embodiments, the drill bit 820 can be cannulated such that a material can be delivered through the drill bit 820 directly to the weakened bone region 806. According to various embodiments, however, the drill bit 820 can be removed and a syringe 860 can be moved relative to the femur 80.

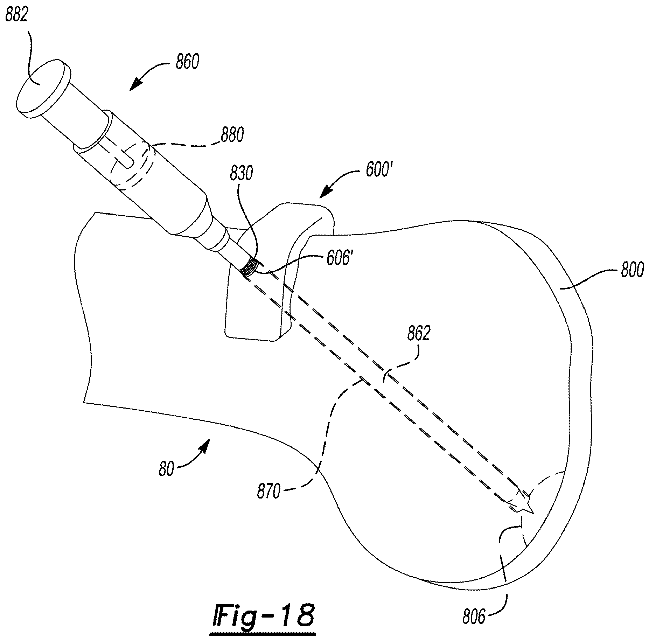

With reference to FIG. 18, the syringe 860 can be positioned relative to the femur 80 such that at least a needle or cannulated member 862 is positioned through the bore 870 that is formed within the femur 80 by the drill bit 820. The bore 870 remains in the femur 80 after the drill bit 820 is withdrawn. The bore 870 is generally a blind bore such that it terminates beneath an external surface of the femur 80. Accordingly, the blind bore 870 does not extend to a surface of the cartilage 800 nor does the drill bit 820 penetrate or contact the cartilage 800. Thus, the cannulated member 862 of the syringe 860 will also not penetrate or contact cartilage 800 during a selected procedure. The bore 870 may also originate at a cartilage free region of the femur 80, or any appropriate bone. A barrel or reservoir 880 can be filled with the selected material that can be ejected through the cannulated member 862 by a plunger mechanism or other pump mechanism 882. A selected treatment, such as a bone void filling material can then be injected into the bone region 806 from the syringe 860.

It is understood, however, that other instruments can be passed through the bore 870 to the weakened bone region 806. Again, the weakened bone region 806 may represent any bone or anatomical feature for which a procedure is appropriate. For example, as discussed above, the weakened bone region 806 can relate to a tumor within the bone, such as within the femur 80. Accordingly, an excising instrument may pass through the bore 870 to the weakened bone region 806 and a biopsy, excision, or other procedure can occur. For example, a biopsy can be obtained of the material within the weakened bone region 806 for a further diagnosis, such as confirmation of a cancerous growth or other growth.

Regardless of the diagnosis of the patient, including the weakened bone region 806, the guide 600' can be designed based upon the plan as illustrated in FIG. 1 to include the guide aperture 606' to guide an instrument relative to a diagnosed region, including the weakened bone region 806. The guiding aperture 606' can allow for guiding an access forming instrument, such as the drill bit 820, and/or a treatment instrument, such as the syringe 860 or a biopsy instrument, as discussed above. The guide 600' can be positioned relative to the femur 80 for various and multiple portions of a procedure to obtain access to a single region for both accessing and/or treating the single region.

The diagnosis region, including the weakened bone region 806 illustrated in FIG. 17, can be accessed due to the patient-specific orientation of the guiding aperture 606' and/or the inner surface 640' of the guide 600' that contacts the femur 80. Accordingly, a procedure can occur that is subchondral and does not engage, contact, or pierce the cartilage 800 of the femur 80. It is understood, in a similar manner, that the guide 600' can be designed to contact any specific bone portion for engaging, treating, or accessing a portion of the bone that is adjacent or near a cartilage or soft tissue portion without contacting or piercing the soft tissue or cartilage portion. Thus, a procedure and/or treatment of the patient can be performed without disturbing the soft tissue, including cartilage, adjacent or on a bone of a patient.

The foregoing discussion discloses and describes merely exemplary arrangements of the present teachings. One skilled in the art will readily recognize from such discussion, and from the accompanying drawings, that various changes, modifications and variations can be made therein without departing from the scope of the invention, which is defined by the appended claims.

## Claims

1. A system for accessing a portion of a femur of a patient relative to a soft tissue on a surface of the femur, comprising:
a guide (600') having a mating patient-specific three-dimensional curved inner surface (640') configured to mate to a portion of the femur (80) relative to a knee joint, the guide having a guiding aperture (606') that when the guide is mated to the femur is directed to a planned location (806) in the femur relative to the soft tissue;
an instrument (820) configured to be guided by the guiding aperture and form a first hole (870) to the planned location into the distal femur bone relative to the knee joint surface through the guiding aperture of the guide; and
a syringe (860) having a member configured to be placed in the formed first hole relative to the soft tissue through the first hole.

2. The system of Claim 1, wherein the instrument is a drill.

3. The system of Claim 1, wherein the mating patient-specific three-dimensional curved inner surface is based on a converted scan data to three dimensional images of the femur of the patient.

4. The system of Claim 1, wherein at least the mating patient-specific three-dimensional curved inner surface of the guide is formed by machining.

5. The system of Claim 1, wherein the syringe is configured to inject a selected material through the member into the first hole in the femur to place a material at a selected location.

6. The system of Claim 5, wherein the member is a needle.

7. The system of Claim 5, wherein the selected material is a bone void filling material.

8. The system of Claim 1, further comprising:
a resection guide configured to guide a cutting blade configured to resect the femur.

## Patentansprüche

1. System für den Zugang zu einem Teil eines Femurs eines Patienten relativ zu einem Weichgewebe auf einer Oberfläche des Femurs, umfassend:
eine Führung (600') mit einer passenden patientenspezifischen dreidimensionalen gekrümmten Innenfläche (640'), die konfiguriert ist, um zu einem Teil des Femurs (80) relativ zu einem Kniegelenk zu passen, wobei die Führung eine Führungsöffnung (606') aufweist, die, wenn die Führung am Femur angepasst ist, einer vorgesehenen Stelle (806) im Femur relativ zum Weichgewebe zugeführt wird;
ein Instrument (820), das konfiguriert ist, um von der Führungsöffnung geführt zu werden und ein erstes Loch (870) an der vorgesehenen Stelle im distalen Femurknochen relativ zur Kniegelenkoberfläche durch die Führungsöffnung der Führung herzustellen; und
eine Spritze (860) mit einem Element, das konfiguriert ist, um im hergestellten ersten Loch relativ zum Weichgewebe durch das erste Loch platziert zu werden.

2. System nach Anspruch 1, wobei das Instrument ein Bohrer ist.

3. System nach Anspruch 1, wobei die passende patientenspezifische dreidimensionale gekrümmte Innenfläche auf in dreidimensionale Bilder des Femurs des Patienten umwandelten Scandaten basiert.

4. System nach Anspruch 1, wobei zumindest die passende patientenspezifische dreidimensionale gekrümmte Innenfläche der Führung durch Bearbeitung hergestellt wird.

5. System nach Anspruch 1, wobei die Spritze konfiguriert ist, um ein ausgewähltes Material durch das Element in das erste Loch im Femur zu injizieren, um ein Material an einer ausgewählten Stelle zu platzieren.

6. System nach Anspruch 5, wobei das Element eine Nadel ist.

7. System nach Anspruch 5, wobei das ausgewählte Material ein Knochenhohlraum-Füllmaterial ist.

8. System nach Anspruch 1, ferner umfassend:
eine Resektionsführung, die konfiguriert ist, um ein für die Resektion des Femurs konfiguriertes Schneidmesser zu führen.

## Revendications

1. Système d'accès à une partie d'un fémur d'un patient par rapport à un tissu mou sur une surface du fémur, comprenant :
un guide (600') ayant une surface interne incurvée tridimensionnelle d'accouplement spécifique à un patient (640') conçue pour s'accoupler à une partie du fémur (80) par rapport à une articulation de genou, le guide ayant une ouverture de guidage (606') qui, lorsque le guide est accouplé au fémur, est dirigé vers un emplacement prévu (806) dans le fémur par rapport au tissu mou ;
un instrument (820) conçu pour être guidé par l'ouverture de guidage et former un premier trou (870) à l'emplacement prévu dans l'os distal du fémur par rapport à la surface de l'articulation du genou au travers de l'ouverture de guidage du guide ; et
une seringue (860) ayant un élément conçu pour être placé dans le premier trou formé par rapport au tissu mou au travers du premier trou.

2. Système selon la revendication 1, dans lequel l'instrument est un foret.

3. Système selon la revendication 1, dans lequel la surface interne incurvée tridimensionnelle d'accouplement spécifique à un patient est basée sur des données de balayage converties en images tridimensionnelles du fémur du patient.

4. Système selon la revendication 1, dans lequel au moins la surface interne incurvée tridimensionnelle d'accouplement spécifique à un patient du guide est formée par usinage.

5. Système selon la revendication 1, dans lequel la seringue est conçue pour injecter un matériau sélectionné au travers de l'élément dans le premier trou dans le fémur pour placer un matériau à un emplacement sélectionné.

6. Système selon la revendication 5, dans lequel l'élément est une aiguille.

7. Système selon la revendication 5, dans lequel le matériau est un matériau de remplissage de vide osseux.

8. Système selon la revendication 1, comprenant en outre :
un guide de résection conçu pour guider une lame de coupe conçue pour réséquer le fémur.
